# EUROPEAN PATENT APPLICATION

(11) **EP 3 531 425 A1**
(43) Date of publication of application: **28.08.2019**
(21) Application number: 16919338.0
(22) Date of filing: 18.10.2016
(51) Int. Cl.: G16H 10/60

(54) **SIMILAR CASE SEARCH PROGRAM, SIMILAR CASE SEARCH DEVICE, AND SIMILAR CASE SEARCH METHOD**

(71) Applicant: Fujitsu Limited, Kawasaki-shi, Kanagawa 211-8588 (JP)
(72) Inventor: KATSUDA, Tadaaki, Kawasaki-shi Kanagawa 211-8588 (JP)
(74) Representative: Hoffmann Eitle
(86) International application number: PCT/JP2016/080850
(87) International publication number: WO 2018/073895

(57) **Abstract**

This enables acquisition of a highly accurate similar case analysis result.

A storing unit (11) stores analysis histories (13a, 13b, ...) each including an analysis result and variable statistical information. The analysis result (14a) indicates a pathological progress stage or a treatment effect analyzed concerning patient data (17). The variable statistical information (15a) indicates values of variables included in patient data (18a, 18b) to which reference is made in the analysis of the pathological progress stage or the treatment effect. A processing unit (12) calculates scores (16a, 16b, ...) of the analysis histories (13a, 13b, ...) based on comparison of values of variables included in patient data (19) and the distributions indicated by the variable statistical information of the respective analysis histories (13a, 13b, ...). The processing unit (12) outputs an analysis result of at least one of the analysis histories (13a, 13b, ...) according to the scores (16a, 16b, ...).

## Description

### Technical Field

The present invention relates to a similar case retrieval program, a similar case retrieval apparatus, and a similar case retrieval method.

### Background Art

In the medical field, an information processing system that assists doctors to treat patients has been used more and more along with the development of an information processing technique. One conceivable example of the information processing system is an information processing system that registers, in a database, clinical information indicating measured test values, adopted treatment methods, and the like for various patients and retrieves, from the database, the clinical information for other patients similar to the clinical information of a target patient as similar cases. It is possible to assist a doctor to make decision for the target patient such as prediction of the progress of a disease state and selection of a treatment method.

For example, there has been proposed a clinical pathway operation support system that generates and presents a clinical pathway, which is a standard medical treatment process. The proposed clinical pathway operation support system classifies various cases registered in a database into a plurality of case groups and generates a clinical pathway for each of the case groups. The clinical pathway operation support system retrieves a case group similar to the clinical information of a target patient and presents the clinical pathway generated for the retrieved case group.

For example, there has been proposed a similar case retrieval apparatus that achieves improvement in retrieval accuracy of similar cases. The proposed similar case retrieval apparatus determines, according to a first collation reference, similarity between an input case input by a user and cases registered in a database and extracts a set of similar cases similar to the input case. The similar case retrieval apparatus calculates statistical information of characteristics concerning the extracted similar cases and presents the extracted similar cases and the statistical information to the user. The similar case retrieval apparatus receives an input of a second collation reference from the user and narrows down the similar cases according to the second collation reference.

For example, there has been proposed a medical treatment plan support system that supports, based on medical images, decision of a medical treatment plan of a target patient. The proposed medical treatment plan support system retrieves similar cases similar to clinical information of the target patient and extracts, from a database, medical images captured in the similar cases. The medical treatment plan support system generates, based on the extracted medical images of the similar cases, a predicted medical image indicating a prediction of a change of a disease part of the target patient and displays the predicted medical image.

### Citation List

### Patent Literature

PTL 1: Japanese Laid-open Patent Publication No. 2003-331055
PTL 2: Japanese Laid-open Patent Publication No. 2008-47154
PTL 3: Japanese Laid-open Patent Publication No. 2011-508331
PTL 4: Japanese Laid-open Patent Publication No. 2014-233611

### Summary of Invention

### Technical Problem

The information processing system retrieving similar cases from the database and analyzing the similar cases is useful in assisting the doctor to make medical treatment of the target patient. However, if the database is created for each of medical institutions, in some case, many cases are accumulated in a certain medical institution (for example, a large hospital) but few cases are accumulated in another medical institution (for example, a small clinic). If cases accumulated in the database are few, it is likely that accuracy of a similar case analysis is deteriorated and usefulness of an analysis result decreases. On the other hand, it is sometimes undesirable from the viewpoint of personal information protection and security to register clinical information itself of a patient treated in a certain medical institution in a database present on the outside of the medical institution and share the clinical information.

In one aspect, an object of the present invention is to provide a similar case retrieval program, a similar case retrieval apparatus, and a similar case retrieval method that make it possible to acquire a highly accurate similar case analysis result.

### Solution to Problem

In one aspect, there is provided a similar case retrieval program causing a computer to execute the following processing comprising: acquiring a plurality of analysis histories each including an analysis result indicating a pathological progress stage or a treatment effect analyzed concerning patient data of a first patient and variable statistical information indicating a distribution of values of a variable included in patient data of a plurality of second patients to which reference is made in the analysis of the pathological progress stage or the treatment effect; receiving designation of patient data of a third patient; and calculating scores of the respective analysis histories based on comparison of values of the variable included in the patient data of the third patient and the distributions of the values of the variable indicated by the variable statistical information of the respective analysis histories, and outputting a result of the analysis of at least one of the plurality of analysis histories according to the scores.

In one aspect, there is provided a similar case retrieval apparatus including a storing unit and a processing unit. In one aspect, there is provided a similar case retrieval method executed by a computer.

### Advantageous Effects of Invention

In one aspect, it is possible to acquire a highly accurate similar case analysis result.

The above and other objects, features, and advantages of the present invention will be clarified by the following explanation related to the accompanying drawings representing preferred forms of implementation as examples of the present invention.

### Brief Description of Drawings

FIG. 1 is a diagram illustrating a similar case retrieval apparatus in a first embodiment.
FIG. 2 is a diagram illustrating an example of an information processing system in a second embodiment.
FIG. 3 is a block diagram illustrating a hardware example of a server apparatus.
FIG. 4 is a block diagram illustrating a function example of the server apparatus.
FIG. 5 is a diagram illustrating an example of a clinical information table.
FIG. 6 is a diagram illustrating an example of a related variable table.
FIG. 7 is a diagram illustrating an example of a pathological progress prediction table.
FIG. 8 is a diagram illustrating an example of a case statistics table.
FIG. 9 is a diagram illustrating a pathological progress graph.
FIG. 10 is a diagram illustrating an example of a treatment effect prediction table.
FIG. 11 is a diagram illustrating an example of a case-by-treatment statistics table.
FIG. 12 is a diagram illustrating an example of a treatment effect graph.
FIG. 13 is a diagram illustrating an example of a retrieval result screen.
FIG. 14 is a flowchart illustrating a procedure example of similar case retrieval.
FIG. 15 is a flowchart (continuation) illustrating the procedure example of the similar case retrieval.
FIG. 16 is a flowchart illustrating a procedure example of analysis result reuse.

### Description of Embodiments

Embodiments are explained below with reference to the drawings.

### [First Embodiment]

A first embodiment is explained.

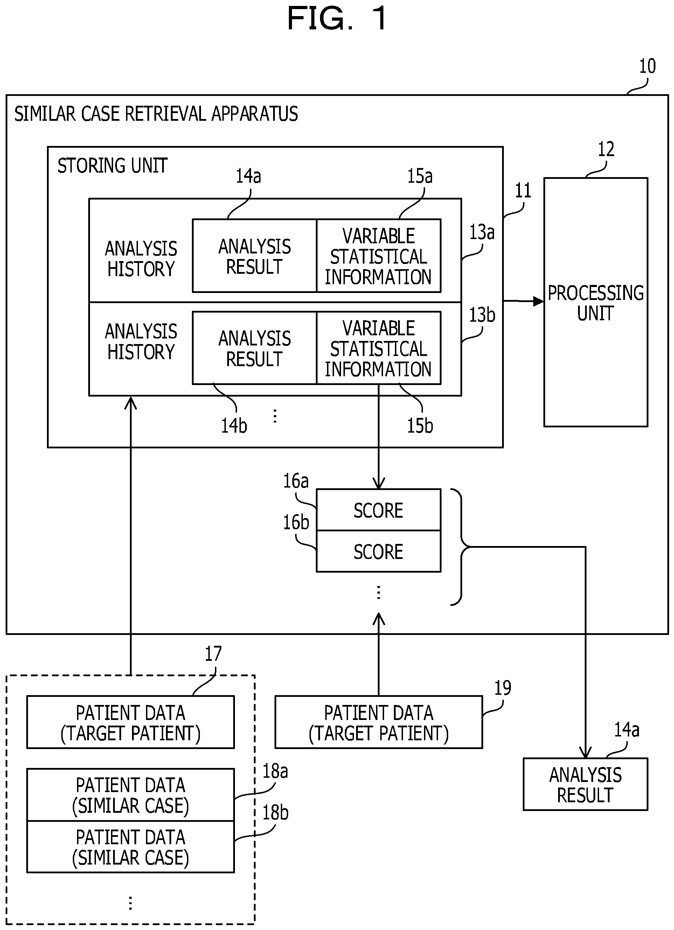

FIG. 1 is a diagram illustrating an example of a similar case retrieval apparatus in the first embodiment.

A similar case retrieval apparatus 10 in the first embodiment is used in an information processing system that assists a doctor to make medical treatment of a patient. In particular, the similar case retrieval apparatus 10 makes it possible to utilize knowledge obtained by referring to patient data of other patients similar to patient data of a target patient.

For example, the similar case retrieval apparatus 10 is used in scenes described below. In a medical institution having many accumulated cases such as a large hospital, it is possible to retrieve, from a database, patient data (similar cases) of other patients similar to patient data of a target patient and obtain an analysis result useful for medical treatment of the target patient referring to the retrieved similar cases. The analysis result sometimes includes information concerning pathological progress prediction of the target patient and information concerning treatment effect prediction in the case in which a certain treatment method is selected. However, in a medical institution having few accumulated cases such as a small clinic, it is likely that accuracy of such a similar case analysis is deteriorated and usefulness of an analysis result decreases. On the other hand, it is sometimes difficult from the viewpoint of personal information protection and security to share patient data itself created in a certain medical institution with other medical institutions.

Therefore, the similar case retrieval apparatus 10 does not share patient data itself and makes it possible to share an analysis history of a similar case analysis performed in a certain medical institution with other medical institutions. The similar case retrieval apparatus 10 makes it possible to retrieve an analysis history matching patient data of a target patient out of shared analysis histories and refer to the analysis history even in a medical institution having few accumulated cases.

The similar case retrieval apparatus 10 includes a storing unit 11 and a processing unit 12. The storing unit 11 may be a volatile semiconductor memory such as a RAM (Random Access Memory) or may be a nonvolatile storage such as a HDD (Hard Disk Drive) or a flash memory. The processing unit 12 is a processor such as a CPU (Central Processing Unit) or a DSP (Digital Signal Processor). However, the processing unit 12 may include an electronic circuit for a specific use such as an ASIC (Application Specific Integrated Circuit) or a FPGA (Field Programmable Gate Array). The processor executes programs stored in a memory such as a RAM. The programs include a similar case retrieval program for causing a computer to execute processing explained below. A set of a plurality of processors is sometimes referred to as "multiprocessor" or "processor".

The storing unit 11 stores a plurality of analysis histories including analysis histories 13a and 13b. The plurality of analysis histories each include an analysis result and variable statistical information. The analysis result indicates a pathological progress stage or a treatment effect analyzed concerning patient data of a target patient. The variable statistical information indicates a distribution of values of a variable included in patient data of other patients to which reference is made when the pathological progress stage or the treatment effect are analyzed. The variable statistical information may indicate a maximum, a minimum, an average, a median, a standard deviation, and the like of the values of the variable. When the variable takes a limited number of discrete values, the variable statistical information may indicate an appearance rate of each value. The analysis history 13a includes an analysis result 14a and variable statistical information 15a. The analysis history 13b includes an analysis result 14b and variable statistical information 15b.

The plurality of analysis histories stored in the storing unit 11 are acquired from the outside of the similar case retrieval apparatus 10. For example, these analysis histories are generated by an apparatus of a medical institution having many accumulated cases such as a large hospital. As an example, the analysis history 13a is generated as explained below.

In a certain medical institution, from a database retained by the medical institution, patient data 18a and 18b and the like are retrieved as similar cases similar to patient data 17 of a target patient. The patient data 17, 18a, and 18b each include values of variables. The variables may include attribute items of patients such as age and sex and test items such as a blood glucose level and a blood pressure. The patient data 18a and 18b are retrieved based on similarity of the values of the variables between the patient data 18a and 18b and the patient data 17. The analysis result 14a is generated referring to the patient data 18a and 18b and the like in the medical institution that retains the database. The variable statistical information 15a is generated by performing statistical processing of the values of the variables included in the patient data 18a and 18b and the like in the medical institution that retains the database. The analysis history 13a does not include contents themselves of the patient data 17, 18a, and 18b.

The processing unit 12 receives designation of patient data 19 of another target patient. The patient data 19 is designated from a medical institution different from the medical institution that generates the analysis histories 13a and 13b, for example, designated from a medical institution having few accumulated cases such as a small clinic. The similar case retrieval apparatus 10 may be set on the outside of the medical institution that designates the patient data 19 or may be owned by the medical institution that designates the patient data 19. The similar case retrieval apparatus 10 receives the patient data 19 from, for example, an apparatus owned by the small clinic.

Like the patient data 17, 18a, 18b, and the like, the patient data 19 includes values of variables. The processing unit 12 compares the values of the variables of the patient data 19 and distributions indicated by the variable statistical information of each of the analysis histories stored in the storing unit 11. For example, the processing unit 12 determines whether the value of each of the variables of the patient data 19 is within a range of the corresponding distribution indicated by each piece of the variable statistical information. The processing unit 12 calculates scores of the analysis histories based on a result of the comparison. A score 16a is calculated for the analysis history 13a. A score 16b is calculated for the analysis history 13b.

The processing unit 12 selects at least one of the plurality of analysis histories stored in the storing unit 11 according to the calculated scores and outputs an analysis result included in the selected analysis history. For example, the processing unit 12 outputs analysis results of the analysis histories having scores equal to or larger than a threshold or analysis results of a predetermined number of the analysis histories from the analysis history having the highest score. When a score of the analysis history 13a is the highest, at least the analysis result 14a is output. The output analysis results are transmitted to, for example, as a response to the patient data 19, an apparatus that designates the patient data 19.

With the similar case retrieval apparatus 10 in the first embodiment, variable statistical information indicating characteristics of similar cases to which reference is made in a similar case analysis in the past is added to an analysis result generated in the similar case analysis in the past and is accumulated as an analysis history. The patient data of the target patient and the variable statistical information are compared. An analysis result in the past matching the target patient is extracted.

Consequently, even if sufficient cases are not accumulated in a database at hand, it is possible to acquire highly accurate analysis result. For example, a medical institution having few accumulated cases is capable of acquiring an analysis result matching another target patient among analysis results of similar case analyses performed in a medical institution having many accumulated cases. Each analysis history does not include patient data itself of a target patient and patient data itself of similar cases used in generating the analysis history. Accordingly, patient data itself created in a certain medical institution does not have to be provided to the outside of the medical institution. It is possible to improve personal information protection and security.

### [Second Embodiment]

A second embodiment is explained.

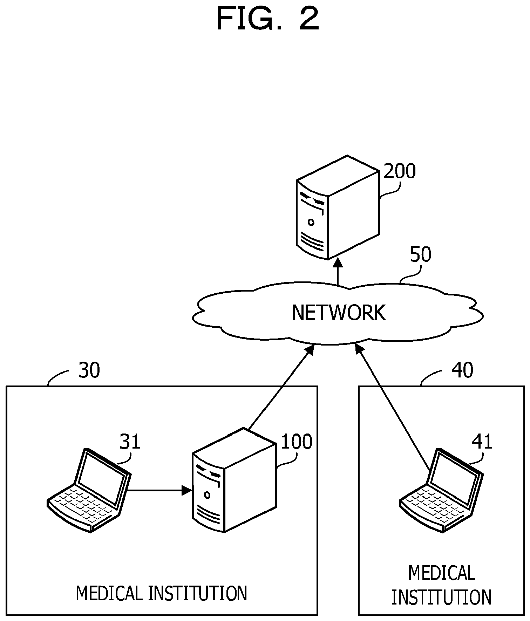

FIG. 2 is a diagram illustrating an example of an information processing system in the second embodiment.

The information processing system in the second embodiment is an information processing system for medical use that assists a doctor to make medical treatment of a patient. This information processing system makes it possible to share useful information among a plurality of medical institutions while considering personal information protection and security.

The information processing system in the second embodiment includes terminal apparatuses 31 and 41 and server apparatuses 100 and 200. The terminal apparatus 31 and the server apparatus 100 are set in a medical institution 30. The terminal apparatus 41 is set in a medical institution 40. The server apparatus 200 is set in a facility such as a data center on the outside of the medical institutions 30 and 40. The terminal apparatus 41 and the server apparatus 100 may access the server apparatus 200 via a network 50. The network 50 is a wide area data network such as the Internet.

The medical institution 30 is a medical institution that retains clinical information of a large number of patients such as a large hospital. The terminal apparatus 31 is a terminal apparatus used by a medical practitioner such as a doctor in the medical institution 30 and is, for example, a client computer. The terminal apparatus 31 may access the server apparatus 100 via a local network of the medical institution 30. The server apparatus 100 is a server apparatus that manages a database having stored therein clinical information of patients who are receiving medical treatment in the medical institution 30 and is, for example, a server computer.

The server apparatus 100 performs a similar case analysis according to a request from the terminal apparatus 31. In the similar case analysis, the server apparatus 100 receives designation of a target patient from the terminal apparatus 31. Then, the server apparatus 100 retrieves, from the database of the server apparatus 100, as similar cases, clinical information of other patients similar to clinical information of the target patient. The server apparatus 100 refers to the similar cases and predicts a pathological progress and a treatment effect in future of the target patient and provides a result of the prediction to the terminal apparatus 31. Consequently, it is possible to assist decision making such as selection of a treatment method for the target patient. Every time the server apparatus 100 performs the similar case analysis, the server apparatus 100 transmits a pathological progress prediction result and a treatment effect prediction result to the server apparatus 200. Communication between the server apparatus 100 and the server apparatus 200 may be protected by an encryption processing technique.

The medical institution 40 is a medical institution such as a small clinic that retains clinical information of a small number of patients compare with the medical institution 30. The terminal apparatus 41 is a terminal apparatus used by a medical practitioner such as a doctor in the medical institution 40 and is, for example, a client computer. The terminal apparatus 41 stores clinical information of patients who are receiving medical treatment in the medical institution 40. However, as in the medical institution 30, a server apparatus may manage a database of clinical information.

Since clinical information accumulated in the medical institution 40 is little, even if the same similar case analysis as the similar case analysis in the medical institution 30 is performed using only the clinical information retained by the medical institution 40, it is likely that accuracy of a pathological progress prediction result and a treatment effect prediction result is deteriorated. Therefore, the terminal apparatus 41 acquires, from the server apparatus 200, a pathological progress prediction result and a treatment effect prediction result highly likely to match a target patient who is receiving medical treatment in the medical institution 40. That is, the information treatment system in the second embodiment makes it possible to reuse, via the server apparatus 200, the pathological progress prediction result and the treatment prediction result generated by the medical institution 30. Consequently, it is possible to assist decision making such as selection of a treatment method for the target patient in the medical institution 40.

Note that the server apparatus 200 corresponds to the similar case retrieval apparatus 10 in the first embodiment. "Clinical information" explained in the second embodiment corresponds to the patient data in the first embodiment.

Hardware and software of the server apparatuses 100 and 200 are explained.

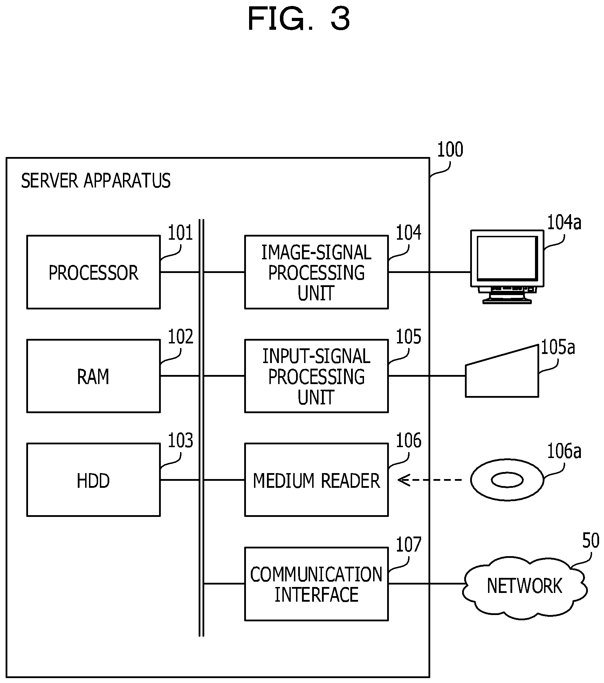

FIG. 3 is a block diagram illustrating a hardware example of the server apparatus.

The server apparatus 100 includes a CPU 101, a RAM 102, a HDD 103, an image-signal processing unit 104, an input-signal processing unit 105, a medium reader 106, and a communication interface 107. The units included in the server apparatus 100 are connected to a bus.

The CPU 101 is a processor including an arithmetic circuit that executes a command of a program. The CPU 101 loads at least a part of programs and data stored in the HDD 103 to the RAM 102 and executes the program. Note that the CPU 101 may include a plurality of processor cores. The server apparatus 100 may include a plurality of processors and may execute processing explained below in parallel using the plurality of processors or processor cores. A set of the plurality of processors is sometimes referred to as "multiprocessor" or "processor".

The RAM 102 is a volatile semiconductor memory that temporarily stores programs executed by the CPU 101 and data used for an arithmetic operation by the CPU 101. Note that the server apparatus 100 may include a memory of a type other than the RAM and may include a plurality of memories.

The HDD 103 is a nonvolatile storage device that stores software programs such as an OS (Operating System), middleware, and application software and data. Note that the server apparatus 100 may include a storage device of another type such as a flash memory or an SSD (Solid State Drive) and may include a plurality of nonvolatile storage devices.

The image-signal processing unit 104 outputs, according to a command from the CPU 101, an image to a display 104a connected to the server apparatus 100. As the display 104a, displays of various types such as a CRT (Cathode Ray Tube) display, an LCD (Liquid Crystal Display), a plasma display, and an organic EL (OEL: Organic Electro-Luminescence) display may be used.

The input-signal processing unit 105 acquires an input signal from an input device 105a connected to the server apparatus 100 and outputs the input signal to the CPU 101. As the input device 105a, a pointing device such as a mouse, a touch panel, a touch pad, or a trackball, a keyboard, a remote controller, a button switch, and the like may be used. Input devices of a plurality of types may be connected to the server apparatus 100.

The medium reader 106 is a reading device that reads programs and data recorded in a recording medium 106a. As the recording medium 106a, for example, a magnetic disk, an optical disk, an MO (Magneto-Optical disk), and a semiconductor memory may be used. The magnetic disk includes a flexible disk (FD: Flexible Disk) and a HDD. The optical disk includes a CD (Compact Disc) and a DVD (Digital Versatile Disc).

The medium reader 106 copies, for example, the programs and the data read from the recording medium 106a to other recording media such as the RAM 102 and the HDD 103. The read programs are executed by, for example, the CPU 101. Note that the recording medium 106a may be a portable recording medium and is sometimes used for distribution of the programs and the data. The recording medium 106a and the HDD 103 are sometimes referred to as computer-readable recording media.

The communication interface 107 is an interface that is connected to the network 50 and performs communication with other apparatuses via the network 50. The communication interface 107 is desirably a wired communication interface connected to a communication apparatus such as a switch by a cable. However, a wireless communication interface connected to a base station by a wireless link may be used.

Note that the terminal apparatus 31 and 41 and the server apparatus 200 may be implemented using the same hardware as the hardware of the server apparatus 100. A CPU included in the server apparatus 200 corresponds to the processing unit 12 explained in the first embodiment. A RAM or a HDD included in the server apparatus 200 corresponds to the storing unit 11 explained in the first embodiment.

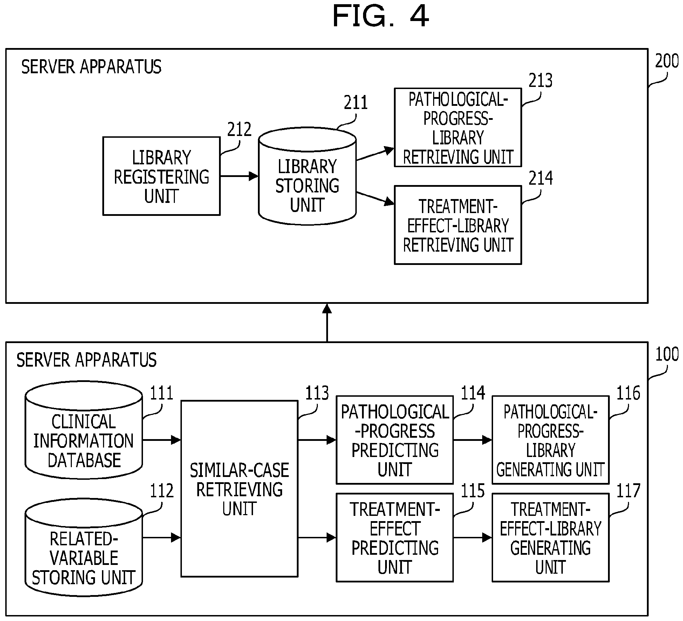

FIG. 4 is a block diagram illustrating a function example of the server apparatus.

The server apparatus 100 includes a clinical information database 111, a related-variable storing unit 112, a similar-case retrieving unit 113, a pathological-progress predicting unit 114, a treatment-effect predicting unit 115, a pathological-progress-library generating unit 116, and a treatment-effect-library generating unit 117. The clinical information database 111 and the related-variable storing unit 112 are implemented using, for example, a storage region secured in the RAM 102 or the HDD 103. The similar-case retrieving unit 113, the pathological-progress predicting unit 114, the treatment-effect predicting unit 115, the pathological-progress-library generating unit 116, and the treatment-effect-library generating unit 117 are implemented using, for example, a program module executed by the CPU 101.

The clinical information database 111 stores clinical information of a plurality of patients. The clinical information is input by a medical practitioner such as a doctor. The clinical information of each patient includes values of a plurality of variables. As the variables, there are attribute items indicating attributes of the patient such as sex and age, test items, values of which are obtained by measurement of a blood glucose level and a blood pressure or diagnosis, items indicating presence or absence of adoption of a treatment method, items indicating a disease state change of the patient such as a relapse-free interval, and the like.

The related-variable storing unit 112 stores related variable information indicating relationship between two variables. The relationship between the two variables may be determined from a correlation between a value taken by one variable and a value taken by the other variable. The relationship is considered to be larger as the correlation is stronger. The other variable having large relationship for a certain variable may be referred to as related variable. Which variable is the related variable for the certain variable may be defined in advance from a characteristic of each variable. The server apparatus 100 may determine which variable is the related variable for the certain variable by analyzing the clinical information stored in the clinical information database 111.

As an algorithm for determining the relationship between the two variables from the clinical information, an appropriate algorithm is selected according to whether each variable takes a limited number of discrete values. As an example, the following algorithms may be used: a chi-square test of Pearson; an exact probability test of Fisher; a Mann-Whitney-Wilcoxon test; a t test of Student; a t test of Welch; a Kruskal-Wallis test; an analysis of variance (ANOVA: Analysis Of Variance); a method of using a rank correlation coefficient of Kendall; a method of using a rank correlation coefficient of Spearman; a method of using a product-moment coefficient of Pearson; and a method of using a MIC (Maximal Information Coefficient). A p value or a correlation coefficient is calculated as an indicator value of the relationship according to the algorithm described above. A set of variables, the indicator value of the correlation of which is equal to or larger than a predetermined threshold or an input threshold, is determined as having large relationship.

The similar-case retrieving unit 113 retrieves, according to a request from the terminal apparatus 31, similar cases similar to clinical information of a target patient from the clinical information database 111. At this time, the similar-case retrieving unit 113 receives designation of a variable together with designation of the target patient from the terminal apparatus 31. The similar-case retrieving unit 113 refers to the related variable information stored in the related-variable storing unit 112, specifies a related variable corresponding to the designated variable, and selects the designated variable and the related variable of the designated variable.

The similar-case retrieving unit 113 uses only a value of the selected variable among values of various variables included in each kind of clinical information and calculates similarity between the value of the variable and the clinical information of the target patient. This is because, since values of miscellaneous variables unrelated to a disease of the target patient are included in each kind of clinical information, if the similarity is calculated using the values of all the variables, it is likely that accuracy of the calculation is deteriorated. A medical practitioner such as a doctor may find similar cases from the viewpoint of a disease in attention by selecting one or more variables related to the disease of the target patient. However, it is complicated for the medical practitioner to explicitly designate all variables used for the calculation of the similarity. Therefore, a related variable related to the designated variable is retrieved. The related variable is also used for the calculation of the similarity.

As the similarity, for example, the product-moment coefficient of Pearson, the rank correlation coefficient of Kendall, the rank correlation coefficient of Spearman, cosine similarity, MIC, and the like may be used. Clinical information, a value of a selected variable value of which is closer to the value of the variable of the target patient, has higher similarity. The similar-case retrieving unit 113 classifies clinical information, similarity of which is equal to or larger than a predetermined threshold or a threshold input from the terminal apparatus 31, into similar cases similar to the clinical information of the target patient. On the other hand, the similar-case retrieving unit 113 classifies other clinical information into comparative cases.

The pathological-progress predicting unit 114 compares and analyzes a set of similar cases and a set of comparative cases to generate a pathological progress prediction result obtained by predicting a pathological progress in future of the target patient. For the prediction of the pathological progress, values of variables concerning disease state changes included in the similar cases and the comparative cases are used. In an example explained below, as the variables concerning the disease state changes, a relapse-free interval, which is the number of days elapses without relapse of a disease from a reference date such as a treatment start date or a complete recovery date, is used.

The pathological progress prediction result includes a pathological progress graph and a predicted prognosis explained below. The pathological progress graph includes a curved line corresponding to similar cases and a curved line corresponding to comparative cases. A coordinate (x, y) on the curved line corresponding to the similar cases indicates that a ratio of cases in which diseases do not relapse at a point in time when x days elapse from the reference date among the similar cases is y%. A coordinate (x, y) on the curved line corresponding to the comparative cases indicates that a ratio of cases in which diseases do not relapse at the point in time when the x days elapse from the reference date among the comparative cases is y%. The two curved lines are monotonous decrease curved lines. The predicted prognosis is a result obtained by predicting whether a prognosis of the target patient is "good", "bad", or "unknown". When the relapse-free intervals of the similar cases are significantly longer than the relapse-free intervals of the comparative cases, the predicted prognosis is "good". When the relapse-free intervals of the similar cases are significantly shorter than the relapse-free intervals of the comparative cases, the predicted prognosis is "bad". When there is no significant difference in the relapse-free intervals between the similar cases and the comparative cases, the predicted prognosis is "unknown". The pathological-progress predicting unit 114 transmits the pathological progress prediction result to the terminal apparatus 31 and causes the terminal apparatus 31 to display the pathological progress prediction result.

The treatment-effect predicting unit 115 receives designation of one or more variables concerning a treatment method from the terminal apparatus 31. Then, the treatment-effect predicting unit 115 determines based on values of the designated variables what kind of a treatment method is adopted by each similar case and classifies the similar cases into a plurality of groups according to treatment methods. Variables of the treatment methods used as classification references are selected by a medical practitioner such as a doctor. The treatment-effect predicting unit 115 compares and analyzes, for each of the groups, the similar cases belonging to the group and the other similar cases to generate a treatment effect prediction result obtained by predicting a pathological progress in future of the target patient in the case in which the treatment method for the similar cases is selected.

For prediction of a treatment effect, values of variables concerning disease state changes included in the similar cases are used. In an example explained below, the relapse-free interval is used. The treatment effect prediction result includes a treatment effect graph and a predicted prognosis for each of treatment methods explained below. The treatment effect graph includes a plurality of curved lines corresponding to a plurality of treatment methods. A coordinate (x, y) on a curved line corresponding to a certain treatment method indicates that a ratio of cases in which diseases do not relapse at a point in time when x days elapse from the reference date among similar cases in which the treatment method is adopted is y%. Each curved line is a monotonous decrease curved line. The predicted prognosis is a result obtained by predicting whether a prognosis of the target patient in the case in which the certain treatment method is adopted is "good", "bad", or "unknown". Comparative cases used for predicting a prognosis corresponding to the certain treatment method are similar cases in which treatment methods other than the certain treatment method are adopted. The treatment-effect predicting unit 115 transmits the treatment effect prediction result to the terminal apparatus 31 and causes the terminal apparatus 31 to display the treatment effect prediction result.

The pathological-progress-library generating unit 116 generates statistical information of the similar case used by the pathological-progress predicting unit 114 in order to generate the pathological progress prediction result. The statistical information indicates a distribution of values among the similar cases concerning each of the variables (the variables designated from the terminal apparatus 31 and the related variables of the variables) used for the calculation of the similarity by the similar-case retrieving unit 113. The distribution of the values of the variable is sometimes represented by a maximum, a minimum, an average, a standard deviation, a median, and the like. The distribution of the values of the variable is sometimes represented by an appearance rate of each value. The pathological-progress-library generating unit 116 associates the pathological progress prediction result generated by the pathological-progress predicting unit 114 and the statistical information of the pathological progress prediction result and transmits the pathological progress prediction result and the statistical information to the server apparatus 200 as information of a pathological progress prediction library.

The treatment-effect-library generating unit 117 generates, for each of groups corresponding to the treatment methods, statistical information of the similar cases used by the treatment-effect predicting unit 115 in order to generate the treatment effect prediction result. The statistical information indicates, concerning each of the variables used for the calculation of the similarity by the similar-case retrieving unit 113, a distribution of values among the similar cases belonging to each group. The treatment-effect-library generating unit 117 associates the treatment effect prediction result generated by the treatment-effect predicting unit 115 and the statistical information of the treatment effect prediction result and transmits the treatment effect prediction result and the statistical information to the server apparatus 200 as information of the treatment effect prediction library.

The server apparatus 200 includes a library storing unit 211, a library registering unit 212, a pathological-progress-library retrieving unit 213, and a treatment-effect-library retrieving unit 214. The library storing unit 211 is implemented using, for example, a storage region secured in a RAM or a HDD included in the server apparatus 200. The library registering unit 212, the pathological-progress-library retrieving unit 213, and the treatment-effect-library retrieving unit 214 are implemented using, for example, a program module executed by a CPU included in the server apparatus 200.

The library storing unit 211 stores the pathological progress prediction library and the treatment effect prediction library. The pathological progress prediction library is a library in which a set of a pathological progress prediction result generated in any medical institution (the medical institution 30 or another medical institution equivalent to the medical institution 30) and statistical information of similar cases to which reference is made during the generation of the pathological progress prediction result is accumulated. The treatment effect prediction library is a library in which a set of a treatment effect prediction result generated in any medical institution and statistical information of similar cases to which reference is made during the generation of the treatment effect prediction result is accumulated. The pathological progress prediction library and the treatment effect prediction library do not include information with which an individual patient may be specified such as clinical information itself of the target patient and similar cases themselves to which reference is made.

The library registering unit 212 receives, from the server apparatus 100 (or a server apparatus of another medical institution), a pathological progress prediction result added with statistical information as a history of a similar case analysis. Then, the library registering unit 212 adds the pathological progress prediction result in the pathological progress prediction library in the library storing unit 211. The library registering unit 212 receives, from the server apparatus 100 (or a server apparatus of another medical institution), a treatment effect prediction result added with statistical information as a history of a similar case analysis. Then, the library registering unit 212 adds the treatment effect prediction result to the treatment effect prediction library in the library storing unit 211.

The pathological-progress-library retrieving unit 213 acquires clinical information of the target patient from the terminal apparatus 41 (or another terminal apparatus). Then, the pathological-progress-library retrieving unit 213 compares the statistical information included in the pathological progress prediction library in the library storing unit 211 and values of variables included in the clinical information of the target patient and calculates a coincidence ratio of each pathological progress prediction result. The pathological-progress-library retrieving unit 213 sorts pathological progress prediction results included in the pathological progress prediction library in the descending order of coincidence ratios and extracts a predetermined number of the pathological progress prediction results from the pathological progress prediction result having the largest coincidence ratio or pathological progress prediction results having coincidence ratios equal to or larger than a threshold. The pathological-progress-library retrieving unit 213 outputs a list of the extracted pathological progress prediction results to the terminal apparatus 41 (or the other terminal apparatus).

The treatment-effect-library retrieving unit 214 acquires clinical information of the target patient from the terminal apparatus 41 (or another terminal apparatus). Then, the treatment-effect-library retrieving unit 214 compares the statistical information included in the treatment effect prediction library in the library storing unit 211 and values of variables included in the clinical information of the target patient and calculates a coincidence ratio of each treatment effect prediction result. The treatment-effect-library retrieving unit 214 sorts treatment effect prediction results included in the treatment effect prediction library in the descending order of coincidence ratios and extracts a predetermined number of the treatment effect prediction results from the treatment effect prediction result having the largest coincidence ratio or treatment effect prediction results having coincidence ratios equal to or larger than a threshold. The treatment-effect-library retrieving unit 214 outputs a list of the extracted treatment effect prediction results to the terminal apparatus 41 (or the other terminal apparatus).

Note that the clinical information of the target patient acquired from the terminal apparatus 41 or the like includes only values of variables designated by a medical practitioner such as a doctor in the medical institution 40. On the other hand, the statistical information added to each pathological progress prediction result and treatment effect prediction result indicates only the distributions of variables used for retrieval of similar cases. Therefore, when the coincidence ratios are calculated, values are compared concerning variables present in common in the clinical information of the target patient and the statistical information.

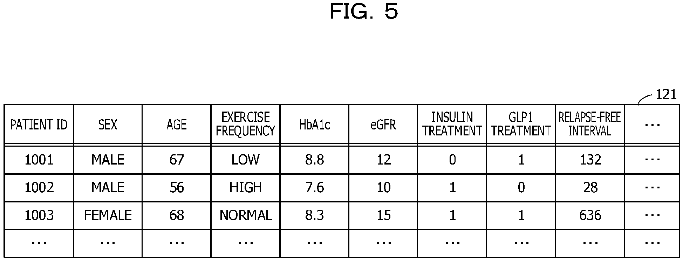

FIG. 5 is a diagram illustrating an example of a clinical information table.

A clinical information table 121 is stored in the clinical information database 111. The clinical information table 121 includes an item of a patient ID and a plurality of variables. The variables include variables indicating attributes of a patient, variables indicating test values obtained by measurement or diagnosis, variables indicating treatment methods, and a variable indicating disease state changes. As an example of the variables indicating the attributes, the clinical information table 121 includes items of sex and age. As an example of the variables indicating the test values, the clinical information table 121 includes items of an exercise frequency, HbA1c, and eGFR. As an example of the variables indicating the treatment methods, the clinical information table 121 includes items of insulin treatment and GLP1 treatment. As an example of the variables indicating the disease state changes, the clinical information table 121 includes an item of a relapse-free interval.

Identification information of the patient is registered in the item of the patient ID. "Male" or "female" is registered in the item of the sex. A nonnegative integer indicating age is registered in the item of the age. "Low", "normal", or "high" indicating a frequency of exercise is registered in the item of the exercise frequency. A nonnegative real number indicating the concentration of hemoglobin combined with glucose in blood is registered in the item of HbA1c. A nonnegative real number indicating a waste excretion ability of the kidney is registered in the item of eGFR.

A flag indicating whether insulin injection is performed as a treatment method is registered in the item of the insulin treatment. "1" indicates that the insulin injection is performed and "0" indicates that the insulin injection is not performed. A flag indicating whether GLP1 injection is performed as the treatment method is registered in the item of the GLP1 treatment. "1" indicates that the GLP1 injection is performed. "0" indicates that the GLP1 injection is not performed. Note that the insulin injection and the GLP1 injection are sometimes used together as the treatment method. The number of days elapsed without relapse of a disease from the reference date is registered in the item of the relapse-free interval.

The various variables included in the clinical information may be classified into a proportional scale, an interval scale, an order scale, and a name scale. When two values (for example, values at different points in time of the same patient) are compared, the proportional scale is a variable meaningful for not only a difference between the values but also for a ratio of the values. Examples of the proportional scale include weight. When two values are compared, the interval scale is a variable meaningful for only a difference between the values and meaningless for a ratio of the values. Examples of the interval scale include body temperature. When two values are compared, the order scale is a variable meaningful for only a magnitude relation between the values and meaningless for a difference and a ratio of the values. Examples of the order scale include a stage indicating a progress degree of a disease. The name scale is a variable indicating a simple division and meaningless for comparing different values. Examples of the name scale include a blood type.

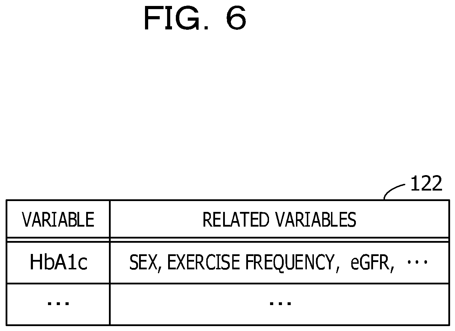

FIG. 6 is a diagram illustrating an example of a related variable table. A related variable table 122 is stored in the related-variable storing unit 112. The related variable table 122 includes items of a variable and related variables. A variable name of a variable sometimes designated from the terminal apparatus 31 when similar cases are retrieved is registered in the item of the variable. Variable names of one or more other variables related to the designated variable are registered in the item of the related variables. As explained above, correspondence between the variable and the related variables may be manually defined based on a characteristic of each variable or the server apparatus 100 may calculate the correspondence by analyzing the clinical information table 121.

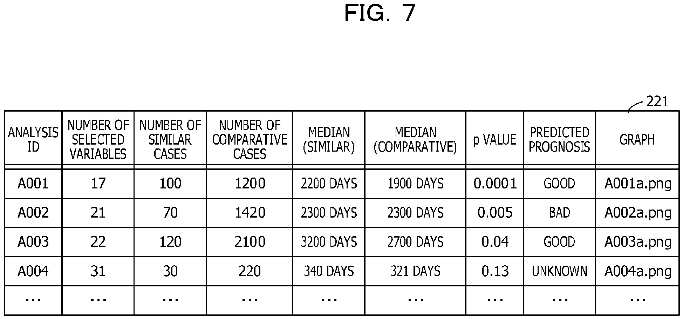

FIG. 7 is a diagram illustrating an example of a pathological progress prediction table.

A pathological progress prediction table 221 is stored in the library storing unit 211. Content of the pathological progress prediction table 221 is equivalent to the pathological progress prediction library. The pathological progress prediction table 221 includes items of an analysis ID, the number of selected variables, the number of similar cases, the number of comparative cases, a median of the similar cases, a median of the comparative cases, a p value, a predicted prognosis, and a graph.

Identification information for identifying an executed similar case analysis is registered in the item of the analysis ID. The number of variables (designated variables and related variables of the designated variables) selected when similar cases are retrieved is registered in the item of the number of selected variables. The number of similar cases determined as being similar to clinical information of the target patient is registered in the item of the number of similar cases. The number of comparative cases, which are cases other than the similar cases (cases determined as being not similar to the clinical information of the target patient), is registered in the item of the number of comparative cases.

A median of the relapse-free intervals of the similar cases is registered in the item of the median of the similar cases. The median of the relapse-free intervals of the similar cases is a relapse-free interval of a similar case in a rank of 50% from the top (for example, fiftieth when the number of similar cases is one hundred) when the similar cases are sorted in the ascending order or the descending order of the relapse-free intervals. A median of the relapse-free intervals of the comparative cases is registered in the item of the median of the comparative cases. The median of the relapse-free intervals of the comparative cases is a relapse-free interval of a comparative case in a rank of 50% from the top when the comparative cases are sorted in the ascending order or the descending order of the relapse-free intervals.

An indicator value indicating a correlation between a group to which a case belongs (whether the case is a similar case or a comparative case) and a relapse-free interval of the case is registered in the item of the p value. The p value is a real number equal to or larger than 0 and equal to or smaller than 1. The p value is smaller as a more significant difference is present in the relapse-free interval between the similar case and the comparative case. Any one of "good", "bad", and "unknown" is registered in the item of the predicted prognosis. An image indicating a pathological progress graph explained below is registered in the item of the graph.

A prognosis may be predicted as explained below. When the p value is equal to or smaller than a threshold (for example, 0.05) and the median of the similar cases is larger than the median of the comparative cases, the prognosis is predicted as "good". When the p value is equal to or smaller than the threshold and the median of the similar cases is equal to or smaller than the median of the comparative cases, the prognosis is predicted as "bad". When the p value is larger than the threshold, the prognosis is predicted as "unknown". That is, when a relapse-free interval of a case similar to a case of the target patient is significantly longer than relapse-free intervals of other cases, the prognosis is good. When the relapse-free interval of the case similar to the case of the target patient is significantly shorter than the relapse-free intervals of the other cases, the prognosis is bad.

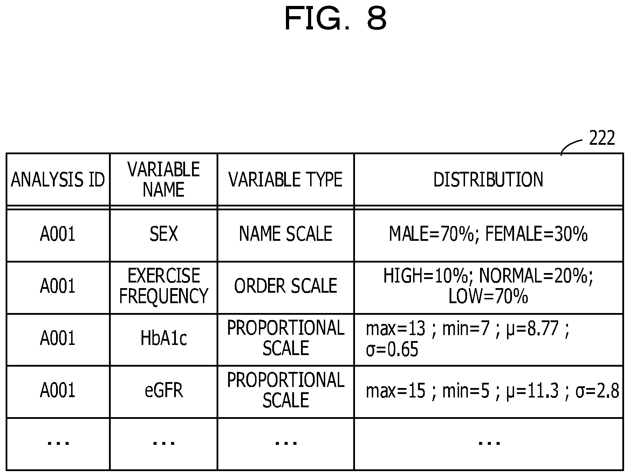

FIG. 8 is a diagram illustrating an example of a case statistics table.

A case statistics table 222 is stored in the library storing unit 211 in association with the pathological progress prediction table 221. The case statistics table 222 includes items of an analysis ID, a variable name, a variable type, and a distribution. An analysis ID corresponding to any record of the pathological progress prediction table 221 is registered in the item of the analysis ID. A name of a variable selected in retrieval of similar cases is registered in the item of the variable name. Any one of a "proportional scale", an "interval scale", an "order scale", and a "name scale" is registered as a type of a variable in the item of the variable type. The variable type may be determined from a variable name. The server apparatus 100 or the server apparatus 200 may retain information indicating a correspondence relation between variable names and variable types.

Information for specifying a distribution of values of each variable included in the similar cases is registered in the item of the distribution. Concerning a variable, a variable type of which is the proportional scale or the interval scale, a maximum, a minimum, an average, a standard deviation, a median, and the like are registered. A first quartile (a value located at 25% from the bottom of the distribution), a third quartile (a value located at 75% from the bottom of the distribution), and the like may be further registered. For example, concerning HbA1c, maximum max=13, minimum min=7, average µ=8.77, and standard deviation σ=0.65 are registered as distribution information. Concerning a variable, a variable type of which is the order scale or the name scale, an appearing ratio of each value is registered. For example, concerning the sex, male=70% and female=30% are registered as the distribution information.

The statistical information registered in the case statistics table 222 is compared with the clinical information of the target patient when the pathological progress prediction library is used. The pathological-progress-library retrieving unit 213 calculates a coincidence ratio for each of variables appearing in common in the statistical information and the clinical information of the target patient.

Concerning a variable, a variable type of which is the proportional scale or the interval scale, the pathological-progress-library retrieving unit 213 determines whether a value of the target patient belongs to a range indicated by the distribution of the statistical information. For example, the pathological-progress-library retrieving unit 213 determines whether the value of the target patient falls between the maximum and the minimum. However, the pathological-progress-library retrieving unit 213 may determine a reference upper limit value and a reference lower limit value using the standard deviation or the like such that an abnormal value in the distribution is removed and determine whether the value of the target patient falls between the reference upper limit value and the reference lower limit value. When the value of the target patient belongs to the range indicated by the distribution of the statistical information, a coincidence ration of the variable is "1". When the value of the target patient does not belong to the range, the coincidence ratio of the variable is "0".

Concerning a variable, a variable type of which is the order scale or the name scale, the pathological-progress-library retrieving unit 213 regards an appearance rate corresponding to the value of the target patient as a coincidence ratio of the variable. For example, when the distribution of the sex is male=70% and female=30% as illustrated in FIG. 8, a coincidence ratio for a target patient whose sex is male is "0.7".

The pathological-progress-library retrieving unit 213 calculates, from coincidence ratios of respective variables appearing in common in the statistical information and the clinical information of the target patient, an overall coincidence ratio between the pathological progress prediction result corresponding to the statistical information and the clinical information of the target patient. A numerator of a calculation formula for calculating the overall coincidence ratio is a total of coincidence ratios of the variables. A denominator of the calculation formula for calculating the overall coincidence ratio is larger one of the number of variables appearing in the statistical information (that is, the number of selected variables described in the pathological progress prediction table 221) and the number of variables included in the clinical information of the target patient.

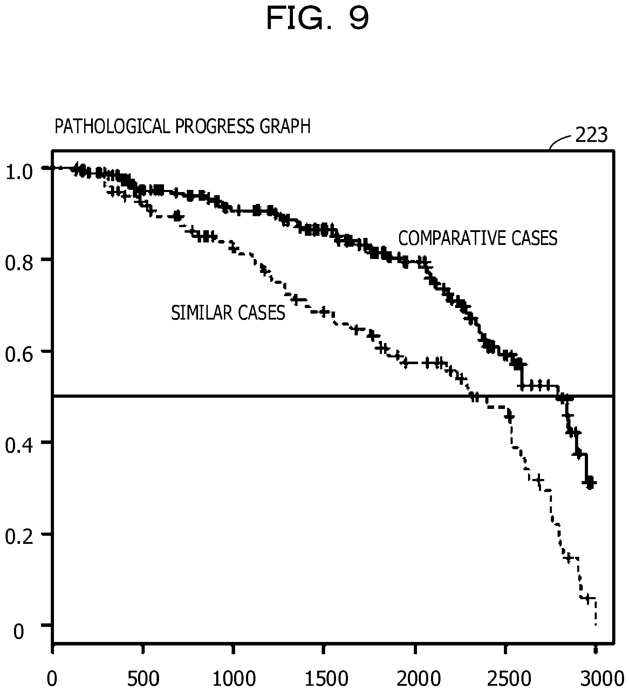

FIG. 9 is a diagram illustrating an example of a pathological progress graph.

A pathological progress graph 223 is an example of a pathological progress graph registered in the item of the graph of the pathological progress prediction table 221. As explained above, the horizontal axis of the pathological progress graph 223 indicates the relapse-free interval and the vertical axis of the pathological progress graph 223 indicates a case ratio. The pathological progress graph 223 includes a monotonous decrease curved line corresponding to similar cases and a monotonous decrease curved line corresponding to comparative cases. A coordinate (x, y) on the curved line corresponding to the similar cases indicates that a ratio of a case, a relapse-free interval of which is x days or more, among the similar cases is y. A coordinate (x, y) on the curved line corresponding to the comparative cases indicates that a ratio of a case, a relapse-free interval of which is x days or more, among the comparative cases is y. In the example illustrated in FIG. 9, the relapse-free intervals of the similar cases are significantly short compared with the relapse-free intervals of the comparative cases.

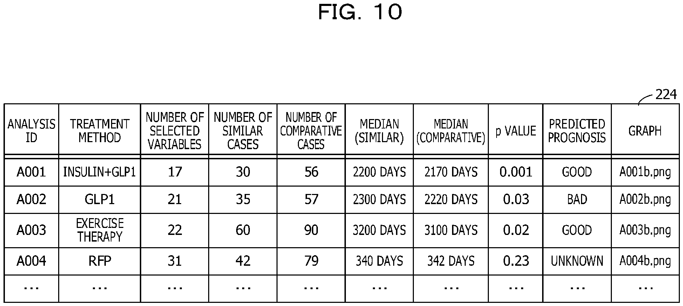

FIG. 10 is a diagram illustrating an example of a treatment effect prediction table.

A treatment effect prediction table 224 is stored in the library storing unit 211. Content of the treatment effect prediction table 224 is equivalent to the treatment effect prediction library. The treatment effect prediction table 224 includes items of an analysis ID, a treatment method, the number of selected variables, the number of similar cases, the number of comparative cases, a median of the similar cases, a median of the comparative cases, a p value, a predicted prognosis, and a graph.

The analysis ID is the same as the analysis ID in the pathological progress prediction table 221. A name of an adopted treatment method, that is, a name of each group (treatment method group) at the time when the similar cases are classified according to the treatment method is registered in the item of the treatment method. The number of selected variables is the same as the number of selected variables in the pathological progress prediction table 221. The number of cases belonging to the treatment method group is registered in the item of the number of similar cases. The number of cases not belonging to the treatment method group (cases belonging to other treatment method groups) among similar cases retrieved from the clinical information of the target patient is registered in the item of the number of comparative cases.

A median of relapse-free intervals of the cases belonging to the treatment method group is registered in the item of the median of the similar cases. A median of relapse-free intervals of the cases belonging to the other treatment method group is registered in the item of the median of the comparative cases. An indicator value indicating a correlation between whether a case belong to the treatment method group and a relapse-free interval is registered in the item of the p value. The p value is smaller as a more significant difference is present in the relapse-free interval between the treatment method group and the other treatment method groups. A prognosis predicted when the treatment method is adopted is registered in the item of the predicted prognosis. An image indicating a treatment effect graph explained below is registered in the item of the graph.

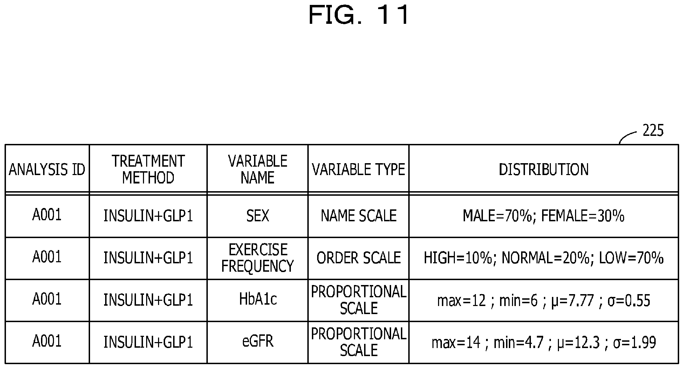

FIG. 11 is a diagram illustrating an example of a case-by-treatment statistics table.

A case-by-treatment statistics table 225 is stored in the library storing unit 211 in association with the treatment effect prediction table 224. The case-by-treatment statistics table 225 includes items of an analysis ID, a treatment method, a variable name, a variable type, and a distribution. An analysis ID corresponding to any record in the treatment effect prediction table 224 is registered in the item of the analysis ID. A treatment method corresponding to any record in the treatment effect prediction table 224 is registered in the item of the treatment method. The variable name and the variable type are the same as the variable name and the variable type in the case statistics table 222.

Information indicating a distribution of values of variables included in a case belonging to the treatment method group is registered in the item of the distribution. Statistical information registered in the case-by-treatment statistics table 225 is compared with the clinical information of the target patient when the treatment effect prediction library is used. The treatment-effect-library retrieving unit 214 calculates a coincidence ratio for each of variables appearing in common in the statistical information and the clinical information of the target patient. Concerning a variable, a variable type of which is the proportional scale or the interval scale, the coincidence ratio is "1" when the value of the target patient belongs to a range indicated by the distribution of the statistical information. The coincidence ratio is "0" when the value of the target patient does not belong to the range. Concerning a variable, a variable type of which is the order scale or the name scale, an appearance rate corresponding to the value of the target patient is regarded as a coincidence ratio of the variable.

The treatment-effect-library retrieving unit 214 calculates, from coincidence ratios of respective variables appearing in common in the statistical information and the clinical information of the target patient, an overall coincidence ratio between a treatment effect prediction result corresponding to the statistical information and the clinical information of the target patient. A numerator of a calculation formula for calculating the overall coincidence ratio is a total of coincidence ratios of the variables. A denominator of the calculation formula for calculating the overall coincidence ratio is larger one of the number of variables appearing in the statistical information (that is, the number of selected variables described in the treatment effect prediction table 224) and the number of variables included in the clinical information of the target patient. Note that one treatment effect prediction result is a prediction result concerning one treatment method group and corresponds to one record of the treatment effect prediction table 224 specified by a set of the analysis ID and the treatment method.

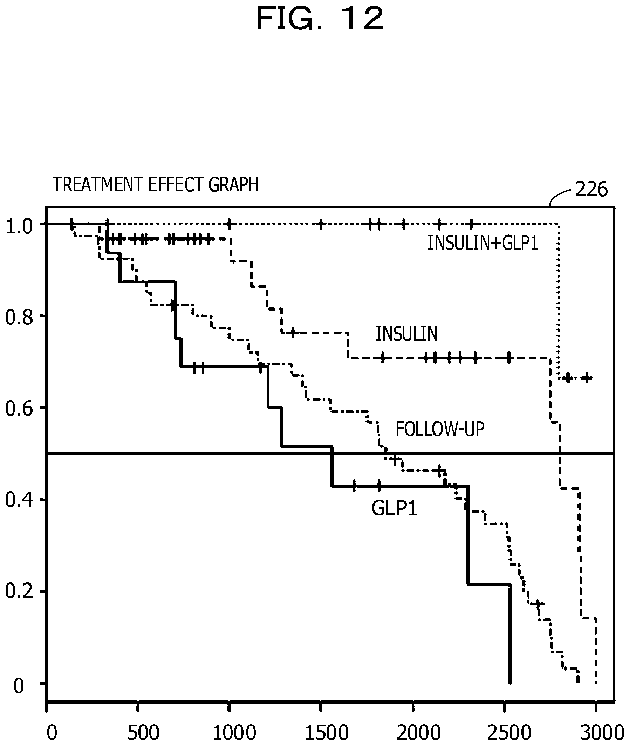

FIG. 12 is a diagram illustrating an example of a treatment effect graph.

A treatment effect graph 226 is an example of a treatment effect graph registered in the item of the graph in the treatment effect prediction table 224. As explained above, the horizontal axis of the treatment effect graph 226 indicates the relapse-free interval and the vertical axis of the treatment effect graph 226 is the case ratio. The treatment effect graph 226 includes a plurality of monotonous decrease curved lines corresponding to a plurality of treatment method groups. A coordinate (x, y) on a curved line corresponding to a certain treatment method group indicates that a ratio of a case, a relapse-free interval of which is x days or more, among cases belonging to the treatment method group is y.

The treatment effect graph 226 illustrated in FIG. 9 indicates effects of a treatment method for using the insulin injection and the GLP1 injection together, a treatment method for performing only the insulin injection, a treatment method for performing only the GLP1 injection, and a treatment method for performing neither the inulin injection nor the GLP1 injection (follow-up). In the example illustrated in FIG. 9, a relapse-free interval in the case in which the insulin injection and the GLP1 injection are used together is the longest. A relapse-free interval in the case in which only the insulin injection is performed is the second longest. A relapse-free interval in the case of the follow-up is the third longest. A relapse-free interval in which only the GLP1 injection is performed is the shortest.

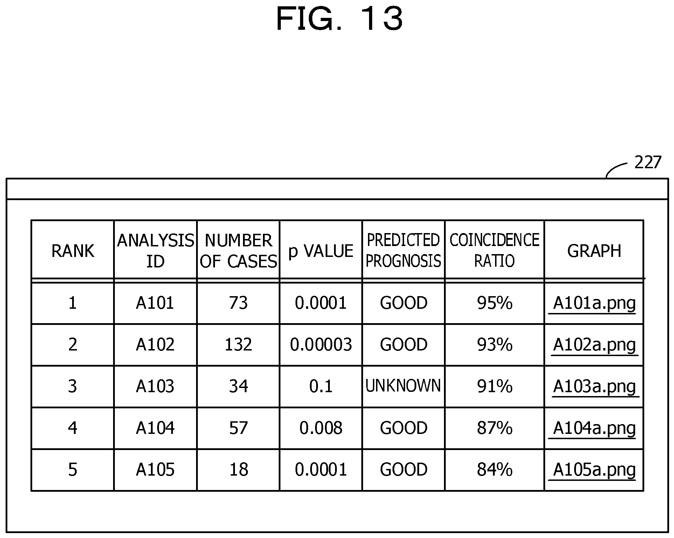

FIG. 13 is a diagram illustrating an example of a retrieval result screen.

As explained above, when receiving the clinical information of the target patient from the terminal apparatus 41, the server apparatus 200 retrieves a pathological progress prediction result having a high coincidence ratio from the pathological progress prediction library and retrieves a treatment effect prediction result having a high coincidence ratio from the treatment effect prediction library. The server apparatus 200 transmits a list of pathological progress prediction results having high coincidence ratios and a list of treatment effect prediction results having high coincidence ratio to the terminal apparatus 41. One or both of the list of the pathological progress prediction results and the list of the treatment effect prediction results are displayed on a display of the terminal apparatus 41.

A retrieval result screen 227 is an example of a screen displayed on the display of the terminal apparatus 41. Only the list of the pathological progress prediction results is displayed. The list of the treatment effect prediction results may be displayed in the same form as the retrieval result screen 227. The terminal apparatus 41 may switch and display the list of the pathological progress prediction results and the list of the treatment effect prediction results according to a user input. The terminal apparatus 41 may individually receive the list of the pathological progress prediction results and the list of the treatment effect prediction results from the server apparatus 200.

A list table including items of a rank, an analysis ID, the number of cases, a p value, a predicted prognosis, a coincidence ratio, and a graph is displayed on the retrieval result screen 227. The rank is order at the time when the pathological progress prediction results are sorted in the descending order of the coincidence ratios. The analysis ID is the analysis ID described in the pathological progress prediction table 221. The number of cases is the number of similar cases described in the pathological progress prediction table 221. The p value and the predicted prognosis are the p value and the predicted prognosis described in the pathological progress prediction table 221. The coincidence ratio is a coincidence ratio calculated by the pathological-progress-library retrieving unit 213. The item of the graph is linked to the pathological progress graph. When this link is selected, the terminal apparatus 41 displays an image of the pathological progress graph acquired from the server apparatus 200 on the display.

Note that, when the treatment effect prediction result is displayed, an item of a treatment method is added to the list table. In that case, the number of cases indicates the number of cases belonging to the treatment method group.

Processing procedures of the server apparatuses 100 and 200 are explained.

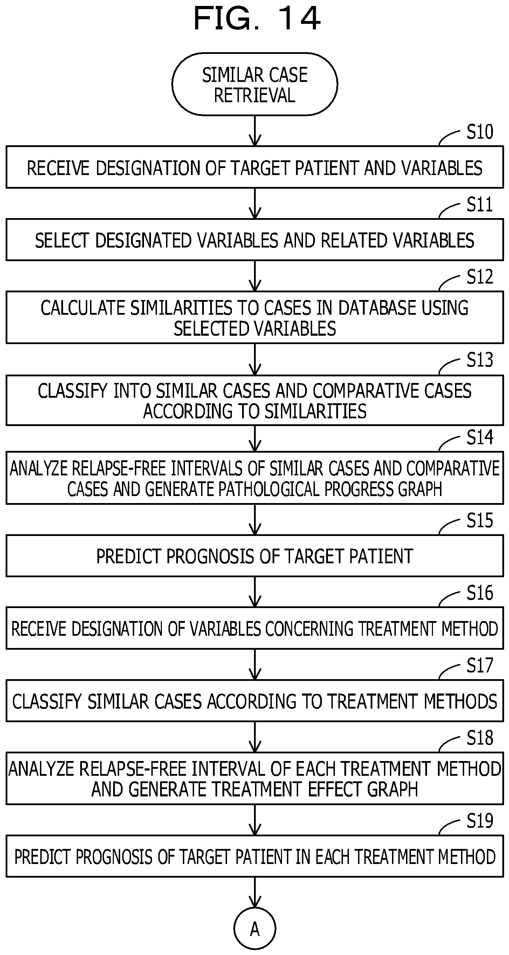

FIG. 14 is a flowchart illustrating a procedure example of the similar case retrieval.

(S10) The similar-case retrieving unit 113 receives designation of a target patient and designation of one or more variables from the terminal apparatus 31. For example, the similar-case retrieving unit 113 acquires, from the terminal apparatus 31, a patient ID of the target patient and variable names of the one or more variables input by a doctor or the like.

(S11) The similar-case retrieving unit 113 retrieves, from the related variable table 122 stored in the related-variable storing unit 112, related variables corresponding to the variables designated in step S10. The similar-case retrieving unit 113 selects the designated variables and the related variables.

(S12) The similar-case retrieving unit 113 extracts, from the clinical information table 121 stored in the clinical information database 111, clinical information of the target patient designated in step S10. The similar-case retrieving unit 113 calculates, based on values of the variables selected in step S11, similarity between the clinical information of the target patient and each of other cases in the clinical information database 111.

(S13) The similar-case retrieving unit 113 classifies the other cases stored in the clinical information database 111 into similar cases and comparative cases according to the similarity calculated in step S12. For example, the similar-case retrieving unit 113 determines that cases having similarity equal to or larger than a threshold are the similar cases and determines that cases having similarity smaller than the threshold are the comparative cases. The similar-case retrieving unit 113 may cause the doctor or the like to input the threshold.

(S14) The pathological-progress predicting unit 114 analyzes relapse-free intervals of the similar cases and relapse-free intervals of the comparative cases and generates the pathological progress graph 223. For example, the pathological-progress predicting unit 114 sorts the similar cases in the ascending order or the descending order of the relapse-free intervals, calculates a ratio y of the similar cases, the relapse-free intervals of which are x days or more, and calculates a curved line corresponding to the similar cases. The pathological-progress predicting unit 114 sorts the comparative cases in the ascending order or the descending order of the relapse-free intervals, calculates a ratio y of the comparative cases, the relapse-free intervals of which are x days or more, and calculates a curved line corresponding to the comparative cases.

(S15) The pathological-progress predicting unit 114 calculates a median of the relapse-free intervals of the similar cases and a median of the relapse-free intervals of the comparison cases. The pathological-progress predicting unit 114 calculates a p value indicates whether a significant difference is present between the relapse-free intervals of the similar cases and the relapse-free intervals of the comparative cases. The pathological-progress predicting unit 114 predicts, based on the median of the similar cases, the median of the comparative cases, and the p value, a prognosis ("good", "bad", or "unknown") of the target patient. The pathological-progress predicting unit 114 transmits a pathological progress prediction result including the pathological progress graph 223 and the predicted prognosis to the terminal apparatus 31.

(S16) The treatment-effect predicting unit 115 receives designation of variables concerning a treatment method from the terminal apparatus 31. For example, the treatment-effect predicting unit 115 acquires variable names input by the doctor or the like.

(S17) The treatment-effect predicting unit 115 classifies, according to values of the variables designated in step S16, the similar cases into a plurality of treatment method groups in which adopted treatment methods are different. Note that a combination of a plurality of prescriptions or follow-up is also recognized as one treatment method.

(S18) The treatment-effect predicting unit 115 analyzes, for each of the treatment method groups, relapse-free intervals of cases belonging to the treatment method group and generates the treatment effect graph 226. The treatment-effect predicting unit 115 sorts, for each of the treatment method groups, the cases in the ascending order or the descending order of the relapse-free intervals, calculates a ratio y of the cases, the relapse-free intervals of which is x days or more, among the cases belonging to the treatment method group, and calculates a curved line corresponding to the treatment method group.

(S19) The treatment-effect predicting unit 115 calculates, for each of the treatment method groups, a median of the relapse-free intervals of the cases belonging to the treatment method group and a median of relapse-free intervals of other similar cases. The treatment-effect predicting unit 115 calculates, for each of the treatment method groups, a p value indicating whether a significant different is present between the relapse-free intervals of the cases belonging to the treatment group and the relapse-free intervals of the other similar cases. The treatment-effect predicting unit 115 predicts, for each of the treatment method groups, based on the median of the cases belonging to the treatment method group, the median of the other similar cases, and the p value, a prognosis of the target patient in the case in which the treatment method is adopted. The treatment-effect predicting unit 115 transmits a treatment effect prediction result including the treatment effect graph 226 and the predicted prognosis to the terminal apparatus 31.

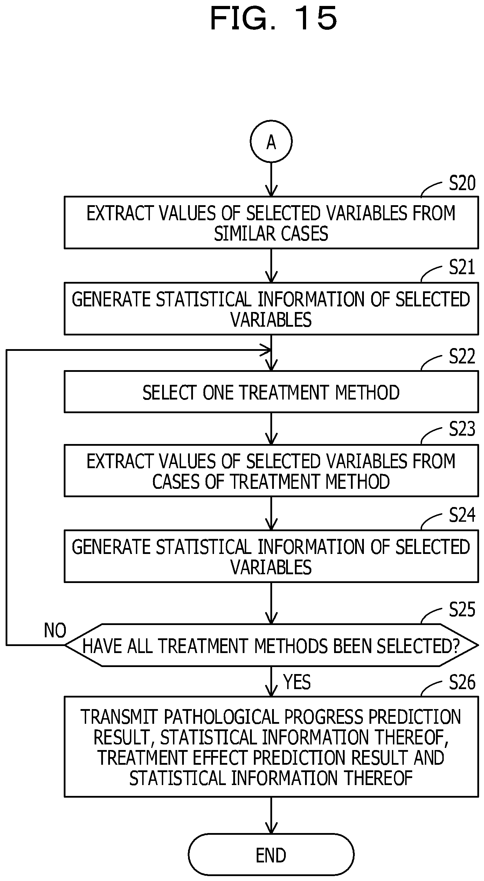

FIG. 15 is a flowchart (continuation) illustrating the procedure example of the similar case retrieval.

(S20) The pathological-progress-library generating unit 116 extracts, from the similar cases in step S13, values of the respective variables selected in step S11.

(S21) The pathological-progress-library generating unit 116 generates information concerning a distribution of the values concerning each of the variables selected in step S11 and sets the information as statistical information corresponding to the pathological progress prediction result. The information concerning the distribution of the values may include an appearance rate of each value. The information concerning the distribution of the values may include statistical indicators such as a maximum, a minimum, an average, a standard deviation, and a median.

(S22) The treatment-effect-library generating unit 117 selects one treatment method.

(S23) The treatment-effect-library generating unit 117 extracts the values of the respective variables selected in step S11 from cases in which the treatment method selected in S22 is adopted (cases belonging to a treatment method group corresponding to the treatment method).

(S24) The treatment-effect-library generating unit 117 generates information concerning distributions of the values of the respective variables selected in step S11 and sets the information as statistical information corresponding to a treatment effect prediction result generated concerning the treatment method selected in step S22. The information concerning the distribution of the values may include an appearance rate of each value. The information concerning the distribution of the values may include statistical indicators such as a maximum, a minimum, an average, a standard deviation, and a median.

(S25) The treatment-effect-library generating unit 117 determines whether all treatment methods have been selected in step S22. When all the treatment methods have been selected, processing proceeds to step S26. When an unselected treatment method is present, the processing proceeds to step S22.

(S26) The pathological-progress-library generating unit 116 associates the pathological progress prediction result in steps S14 and S15 and the statistical information in step S21 and transmits the pathological progress prediction result and the statistical information to the server apparatus 200. The treatment-effect-library generating unit 117 associates the treatment effect prediction result in steps S18 and S19 and the statistical information in step S24 and transmits the treatment effect prediction result and the statistical information to the server apparatus 200.

In the server apparatus 200, the pathological progress prediction result is registered in the pathological progress prediction table 221. The statistical information corresponding to the pathological progress prediction result is registered in the case statistics table 222. The treatment effect prediction result is registered in the treatment effect prediction table 224. The statistical information corresponding to the treatment effect prediction result is registered in the case-by-treatment statistics table 225.

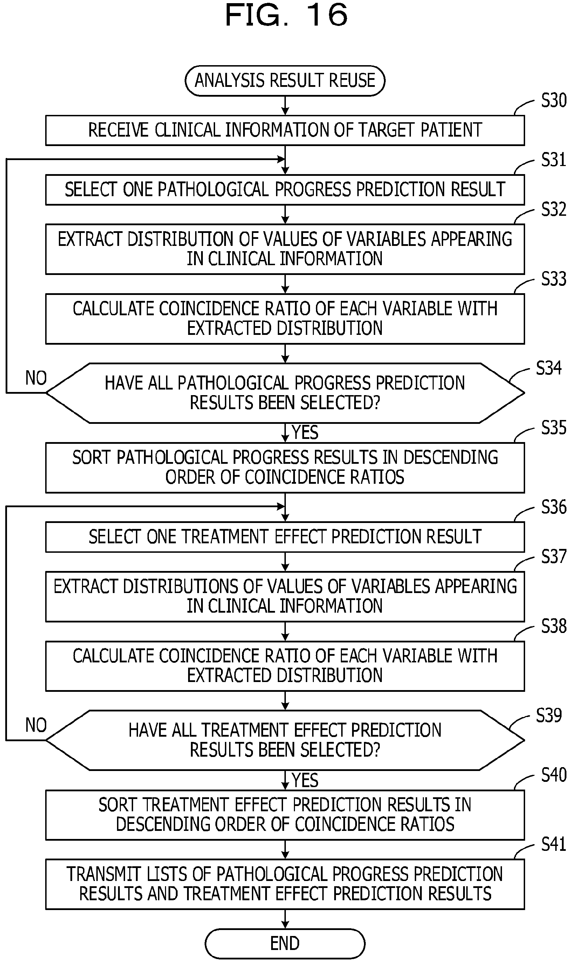

FIG. 16 is a flowchart illustrating a procedure example of analysis result reuse.

(S30) The server apparatus 200 receives clinical information of the target patient from the terminal apparatus 41. Variables appearing in the clinical information, that is, variables used for library retrieval are selected by a medical practitioner such as a doctor who operates the terminal apparatus 41.

(S31) The pathological-progress-library retrieving unit 213 selects one pathological progress prediction result registered in the pathological progress prediction table 221.

(S32) The pathological-progress-library retrieving unit 213 extracts, from the case statistics table 222, information concerning distributions of variables appearing in the clinical information of the target patient among statistical information corresponding to the pathological progress prediction result selected in step S31.

(S33) The pathological-progress-library retrieving unit 213 compares, concerning each of the variables appearing in the clinical information of the target patient, a value included in the clinical information and the information concerning the distribution extracted in step S32 and calculates a coincidence ratio of the variable. The pathological-progress-library retrieving unit 213 divides a total of the coincidence ratios of the variables by a larger one of the number of the variables appearing in the clinical information of the target patient and the number of selected variables indicated by the pathological progress prediction result to calculate an overall coincidence ratio.

(S34) The pathological-progress-library retrieving unit 213 determines whether all pathological progress prediction results have been selected in step S31. When all the pathological progress predication results have been selected, processing proceeds to step S35. When an unselected pathological progress prediction result is present, the processing proceeds to step S31.

(S35) The pathological-progress-library retrieving unit 213 sorts the pathological progress prediction results in the descending order of the coincidence ratios. The pathological-progress-library retrieving unit 213 extracts, as a retrieval result, a predetermined number of the pathological progress prediction results from the pathological progress prediction result having the highest coincidence ratio or the pathological progress prediction results having the coincidence ratios equal to or larger than a threshold. The pathological-progress-library retrieving unit 213 may cause the doctor or the like to input the threshold of the coincidence ratio.

(S36) The treatment-effect-library retrieving unit 214 selects one treatment effect prediction result registered in the treatment effect prediction table 224.

(S37) The treatment-effect-library retrieving unit 214 extracts, from the case-by-treatment statistics table 225, information concerning distributions of the variables appearing in the clinical information of the target patient among statistical information corresponding to the treatment effect prediction result selected in step S36.

(S38) The treatment-effect-library retrieving unit 214 compares, concerning each of the variables appearing in the clinical information of the target patient, a value included in the clinical information and the information concerning the distribution extracted in step S37 and calculates a coincidence ratio of the variable. The treatment-effect-library retrieving unit 214 divides a total of the coincidence ratios of the variables by a larger one of the number of the variables appearing in the clinical information of the target patient and the number of selected variables indicated by the treatment effect prediction result to calculate an overall coincidence ratio.

(S39) The treatment-effect-library retrieving unit 214 determines whether all treatment effect prediction results have been selected in step S36. When all the treatment effect prediction results have been selected, the processing proceeds to step S40. When an unselected treatment effect prediction result is present, the processing proceeds to step S36.

(S40) The treatment-effect-library retrieving unit 214 sorts the treatment effect prediction results in the descending order of the coincidence ratios. The treatment-effect-library retrieving unit 214 extracts, as a retrieval result, a predetermined number of the treatment effect prediction results from the treatment effect prediction result having the highest coincidence ratio or the treatment effect prediction results having the coincidence ratios equal to or larger than a threshold. The treatment-effect-library retrieving unit 214 may cause the doctor or the like to input the threshold of the incidence ratio.

(S41) The pathological-progress-library retrieving unit 213 transmits a list of the pathological progress prediction results extracted in step S35 to the terminal apparatus 41. The treatment-effect-library retrieving unit 214 transmits a list of the treatment effect prediction results extracted in step S40 to the terminal apparatus 41. The list of the pathological progress prediction results and the list of the treatment effect prediction result may be transmitted together or may be individually transmitted according to a request from the terminal apparatus 41.

With the information processing system in the second embodiment, statistical information indicating characteristics of similar cases to which reference is made in generating pathological progress prediction results and treatment effect prediction results for specific target patients in a medical institution such as a large hospital is added to the pathological progress prediction results and the treatment effect prediction results. The pathological progress prediction results and the treatment effect prediction results and the statistical information are registered in a library. Clinical information and statistical information of another target patient are compared with the pathological progress prediction results and the treatment effect prediction results and the statistical information. The pathological progress prediction results and the treatment effect prediction results highly likely to match the other target patient are retrieved from the library. Consequently, even a medical institution such as a small clinic having few accumulated cases is capable of acquiring highly accurate pathological progress prediction results and treatment effect prediction results. The library does not include the clinical information itself of the target patients used during the analysis and the clinical information itself, which is similar cases. Accordingly, even if the library is shared, each medical institution does not have to provide clinical information in the medical institution to the outside. It is possible to improve personal information protection and security.

The above explanation simply indicates the principle of the present invention. Further, a large number of modifications and changes are possible for those skilled in the art. The present invention is not limited to the explained accurate configurations and application examples. All modifications and equivalents corresponding to the present invention are regarded as falling within the scope of the present invention specified by the appended claims and their equivalents. Reference Signs List

- 10: similar case retrieval apparatus
- 11: storing unit
- 12: processing unit
- 13a, 13b: analysis history
- 14a, 14b: analysis results
- 15a, 15b: variable statistical information
- 16a, 16b: score
- 17, 18a, 18b, 19: patient data

## Claims

1. A similar case retrieval program causing a computer to execute processing comprising:
acquiring a plurality of analysis histories each including an analysis result indicating a pathological progress stage or a treatment effect analyzed concerning patient data of a first patient and variable statistical information indicating a distribution of values of a variable included in patient data of a plurality of second patients to which reference is made in the analysis of the pathological progress stage or the treatment effect;
receiving designation of patient data of a third patient; and
calculating scores of the respective analysis histories based on comparison of a value of the variable included in the patient data of the third patient and the distributions of the values of the variable indicated by the variable statistical information of the respective analysis histories, and outputting the analysis result of at least one of the analysis histories according to the scores.

2. The similar case retrieval program according to claim 1, wherein
the patient data of the plurality of second patients and the patient data of the third patient each include values of pluralities of variables, and
the variable statistical information indicates distributions of the values of the respective variables, and
each of the scores is calculated according to how many variables among the pluralities of variables in the patient data of the third patient have values each being within a range indicated by the corresponding distribution of the variable statistical information.

3. The similar case retrieval program according to claim 1, wherein the variable indicated by the variable statistical information is a variable used to determine similarity between the patient data of the first patient and the patient data of each of the plurality of second patients.

4. The similar case retrieval program according to claim 1, wherein the patient data of the plurality of second patients are similar case data in which similarity to the patient data of the first patient is equal to or larger than a threshold.

5. A similar case retrieval apparatus comprising:
a storing unit that stores a plurality of analysis histories each including an analysis result indicating a pathological progress stage or a treatment effect analyzed concerning patient data of a first patient and variable statistical information indicating a distribution of values of a variable included in patient data of a plurality of second patients to which reference is made in the analysis of the pathological progress stage or the treatment effect; and
a processing unit that receives designation of patient data of a third patient, calculates scores of the respective analysis histories based on comparison of a value of the variable included in the patient data of the third patient and the distributions of the values of the variable indicated by the variable statistical information of the respective analysis histories, and outputs the analysis result of at least one of the analysis histories according to the scores.

6. A similar case retrieval method executed by an information processing system, the similar case retrieval method comprising:
retrieving patient data of a plurality of second patients using patient data of a first patient;
generating an analysis result indicating a pathological progress stage or a treatment effect concerning the patient data of the first patient by referring to the patient data of the plurality of second patients and generating variable statistical information indicating a distribution of values of a variable included in the patient data of the plurality of second patients;
storing, in a database, analysis histories in each of which the analysis result and the variable statistical information are associated with each other, to accumulate a plurality of analysis histories in the database;
when patient data of a third patient is designated, calculating scores of the respective analysis histories based on comparison of a value of the variable included in the patient data of the third patient and the distributions of the values of the variable indicated by the variable statistical information of the respective analysis histories; and
outputting the analysis result of at least one of the analysis histories according to the scores.
